# EUROPEAN PATENT SPECIFICATION

(11) **EP 0 733 370 B1**
(45) Date of publication and mention of the grant of the patent: **19.02.2003**
(21) Application number: 93923675.8
(22) Date of filing: 29.10.1993
(51) Int. Cl.: A61K 31/70, A61K 48/00, A61P 19/02

(54) **USE OF A GENE PROMOTER SEQUENCE FOR THE TREATMENT OF RHEUMATIC DISEASES**
VERWENDUNG DER GENSEQUENZ EINES PROMOTORS ZUR BEHANDLUNG RHEUMATISCHER ERKRANKUNGEN
UTILISATION DE LA SEQUENCE D'UN PROMOTEUR DE GENE POUR LE TRAITEMENT DE MALADIES RHEUMATISMALES

(43) Date of publication of application: 25.09.1996
(73) Proprietor: Shiozawa, Shunichi, Kobe-shi, Hyogo 651-22 (JP)
(72) Inventor: Shiozawa, Shunichi, Kobe-shi, Hyogo 651-22 (JP)
(74) Representative: Pett, Christopher Phineas
(86) International application number: JP9301581
(87) International publication number: WO95011684

(56) References cited:
- WO-A-91/06570
- WO-A-91/11535
- WO-A-92/07072
- WO-A-92/18522
- WO-A-93/14768
- TAKAHASHI, H. ET AL: "Analysis of the 5'-upstream promoter region of human involucrin gene: activation by 12-O-tetradecanoylphorbol-13-acetate" THE JOURNAL OF INVESTIGATIVE DERMATOLOGY, vol. 100, no. 1, January 1993 (1993-01), pages 10-15, XP002912488
- ANGEL, P. ET AL: "Phorbol ester inducible genes contain a common cis element recognized by a TPA-modulated trans-acting factor" CELL, vol. 49, 1987, pages 729-739, XP002121076
- BIELINSKA, A. ET AL: "Regulation of gene expression with double-stranded phosphorothioate oligonucleotides" SCIENCE, vol. 247, 1990, pages 997-1000, XP000206516
- ANFOSSI, G. ET AL: "An oligomer complementary to c-myb-encoded mRNA inhibits proliferation of human myeloid leukemia cell lines" PROCEEDINGS OF THENATIONAL ACADEMY OF SCIENCES USA, vol. 86, May 1989 (1989-05), pages 3379-3383, XP000652103
- RITCHLIN, C.T. ET AL: "Potential mechanisms for coordinate gene activation in the rheumatoid synoviocyte: Implications and hypotheses" SPRINGER SEMINARS IN IMMUNOPATHOLOGY, vol. 11, 1989, pages 219-234, XP002121077
- SHIOZAWA, S. ET AL: "Contribution of synovial mesenchymal cells to the pathogenesis of rheumatoid arthritis" SEMINARS IN ARTHRITIS AND RHEUMATISM, vol. 21, no. 4, 1992, pages 267-273, XP002120972
- KUROKI, Y. ET AL: "The contribution of human c-fos DNA to cultured synovial cells: a transfection study" THE JOURNAL OF RHEUMATOLOGY, vol. 20, no. 3, March 1993 (1993-03), pages 422-428, XP002120971
- SHIOZAWA, S. ET AL: "Destructive arthritis without lymphocytic infiltration in H2-c-fos transgenic mice" THE JOURNAL OF IMMUNOLOGY, vol. 148, no. 10, 1992, pages 3100-3104, XP002120973
- KUROKI, Y. ET AL: "Constitutive expression of c-fos gene inhibits type 1 collagen synthesis in transfected osteoblasts" BIOCHEMICAL AND BIOPHYSICAL RESEARCH COMMUNICATIONS, vol. 182, no. 3, 1992, pages 1389-1394, XP002120974
- HOLT, J.T. ET AL: "Inducible production of c-fos antisense RNA inhibits 3T3 cell proliferation" PROCEEDINGS OF THE NATIONAL ACADEMY OF SCIENCES USA, vol. 83, 1986, pages 4794-4798, XP002121078
- NUCLEIC ACIDS RESEARCH; Vol, 21, (No. 11), p. 2715-21, (1993), T. YOSHIDA et al., "Analysis of Fra-2 Gene Expression".
- THE JOURNAL OF BIOLOGICAL CHEMISTRY, Vol. 267, (No. 30), p. 21894-21900, (1992), J. ALAM et al., "Distal AP-1 Binding Sites Mediate Basal Level Enhansment and TPA Induction of the Mouse Heme Oxygenase-1 Gene".

## Description

### TECHNICAL FIELD

The present invention relates to the use of a gene promoter sequence containing TGAGTCA or TGACTCA in the production of a medicament for the treatment of collagen diseases. Such a medicament is useful for the inhibition of the binding of c-Fos protein onto genes thereby inhibiting development of rheumatic diseases.

### BACKGROUND ART

Collagen diseases meaning a group of diseases mainly taking the form of inflammation of connective tissue in a human body has conventionally been known throughout the world as intractable diseases of which it was difficult to clarify the causes and to be successful in therapeutic treatment.

Collagen diseases are believed to include rheumatoid arthritis, rheumatic fever, polymyositis, scleroderma, and the like.

In spite of extensive investigation worldwide, effective therapeutic means have not as yet been found.

Although rheumatoid arthritis, for example, shows a high morbidity rate as 0.3% of the population, it is still one of intractable diseases of which the cause is not as yet known. Therapeutic agents exhibiting a remarkable effect have not as yet been developed, such that the therapeutic level is still on an empirical one. Such current circumstances are considered attributable at least partially to the fact that the present development efforts are not always properly based on results of the most advanced research on the cause of diseases.

In rheumatoid arthritis, proliferation of synovial cells in the joints is abnormally accelerated by any of various inflammatory agents, leading to joint destruction. Conventional research and development efforts of therapeutic agents have however been made from the point of view of inhibiting these inflammatory agents (interrheukine-1, adhesion molecules on the vessel), however an effective one is not as yet available.

However, previous studies have shown that rheumatoid arthritis proceeds from joint inflammation to joint destruction and then joint deformation, and although the cause is unknown, demonstrated the following disease conditions:
(1) Some antigen (bacterium or virus) reaches the joint and evokes inflammation in the joint;
(2) Arthritis becomes chronic;
(3) This leads to joint destruction.

Becoming chronic as mentioned under (2) is due to abnormal acceleration of proliferation of synovial cells in the joint.

Synovial cell proliferation is considered to be closely associated with the increase of substances (cytokine, etc.) that stimulate synovial cells.

The process to rheumatoid arthritis conceived in the present studies on causes of rheumatism and other research and development efforts of a therapeutic agent is that first a local immune reaction is started by an unknown etiologic agent having reached the joint synovial membrane from circulation, then, the infiltration of macrophages, T cells, B cells and neutrophils from blood results in complicated chronic inflammation which finally leads to joint destruction. Important factors participating in this process include specific recognition systems such as T cells which are responsible for immunological memory of specific response to antigen and B cells under the control thereof, as well as nonspecific systems such as cytokines and adhesion molecules broadly associated with chronic inflammation, autoantibodies (rheumatoid factors), and proteases, and the conventional study efforts have been made to inhibit them. Recently in particular, investigators in the world have been competing to inhibit the action of cytokines and adhesion molecules. Current research indicates that, although the immune response system mainly comprising T cells surely constitutes a major portion of the causes of rheumatoid arthritis, what "directly" participate in joint destruction are at least those cells known as nonspecific synovial mesenchymal cells.

Attention given to this mesenchymal cells is noted as a point of view not only toward rheumatoid arthritis, but also toward collagen disease itself.

WO92/18522 discloses compositions comprising an oligonucleotide comprising a first, second and third segment, wherein the first segment, when hybridised with its complement, forms at least one transcription control sequence of at least 6 nucleotides. The AP-1 element (having the sequence 5'-TGAGTCAG-3') is given as one example of such a transcription control sequence.

WO91/11535 discloses oligonucleotide sequences which are useful for inhibiting HIV-1 transcription and refers specifically to AP-1 elements having the sequences TGAGTCAG and C/GTGACTC/AA.

In spite of the progress in research, however, an effective therapeutic agent or an effective therapeutic method has not as yet been established at present regarding collagen diseases including rheumatoid arthritis.

An object of the present invention is therefore to provide a use for producing a medicament that overcomes previous technical limits as described above and is effective for therapy of collagen diseases including rheumatoid arthritis.

### DISCLOSURE OF INVENTION

As means to solve the above-mentioned problems, the present invention provides the use of a gene promoter sequence containing TGAGTCA or TGACTCA in the production of a medicament for the treatment of a collagen disease. Preferably, the collagen disease is rheumatoid arthritis.

### BEST MODE FOR CARRYING OUT THE INVENTION

The present invention mentioned above is basically to prevent expression of causes of diseases, for example, joint destruction and the like by inhibiting synovial mesenchymal cells. More specifically, the present invention aims at achieving successful therapy, for rheumatoid arthritis, for example, by inhibiting synovial mesenchymal cells directly related with joint destruction, accurately but without entering a complicated bush of T cell responses. This concept is unique to the present inventor based on the results of his study on causes of diseases and is unprecedented in the world at present.

More particularly, in arthritic lesions of rheumatoid arthritis, the T cell-centered immune response responsible for immunological memory and the synovial mesenchymal cell "directly" participating in joint destruction are two basically important factors (Shiozawa et al., Ann. Rheum. Dis.51: 869, 1992). The latter is a major component of pannus and produces cytokines such as IL1, IL6 and TNFα that are important in synovial lesion of rheumatoid arthritis (Shiozawa et al., Sem. Arthritis Rheum. 21: 267, 1992). While pannus has a proliferation potency just like a tumor, a joint destruction is induced by synovial mesenchymal cells alone without local infiltration of lymphocyte when actually causing experimental arthritis (antigen-induced arthritis) in H2-c-fos transgenic mice prepared by recombination of c-fos gene, a protooncogene, in the downstream of H-2 promoter (Shiozawa et al., J. Immunol. 148: 3100, 1992). The c-fos gene imparts an unique transformation of synovial cells (transformation from dendritic cell to fibroblastic cell) and a proliferation potency (Kuroki, Shiozawa et al., J. Rheumatol. 20: 422, 1993). This c-fos gene is expressed in a large quantity in rheumatoid arthritis synovial membrane, and continuous expression of the human c-fos gene in osteoblast by the transfection method inhibits syntheses of type I collagen and expressions of mRNA (Kuroki, Shiozawa et al., BBRC 182: 1389, 1992).

These facts suggest that over-expression of c-fos gene not only participates in "direct" joint destruction through stimulation of the proliferation of synovial mesenchymal cells, but also participates, as a cause, in osteoporosis of rheumatoid arthritis.

The present invention was developed on the basis of the findings that c-fos gene activates particularly mesenchymal cells, as a result of examination of the mechanism of action thereof.

More specifically, the present invention is based on a premise that c-Fos protein acts on the AP-1 site in the promoter of pathogenic gene of rheumatoid arthritis, thereby adjusting gene expression. This promoter has a structure represented by the following formula: 
5' - GTG TTA CCC TGA GTC AGA GGA GAA - 3'
3' - AAT GGG ACT CAG TCT CCT CTT GGG - 5
 and the AP-1 site means TGA GTC A underscored in the formula. Of the two strands of the double-stranded DNA, only one is read upon synthesis of protein, and c-Fos protein complexes bind to the both.

In the present invention, therefore, the AP-1 nucleotide comprising TGAGTCA or TGACTCA which is a part of gene promoter as described above is used as an antagonistic inhibitor of the proliferation of mesenchymal cells.

This permit very effective inhibition of the expression of rheumatoid arthritis and the like. It is needless to mention that the AP-1 nucleotide mentioned above may be used in combination with any of various other additional conditions.

The present invention will now be described further in detail by means of examples.

### EXAMPLES

DBA 1/J male mice were subcutaneously immunized with FCA and 200µg of type II collagen twice at a time interval of three weeks, and after two weeks from the initial immunization, 5µg of double-stranded AP-1 nucleotide each was intraperitoneally administered at a rate of twice per week. A nonspecific double-stranded nucleotide was used as a control, and upon three weeks from the completion of the second immunization, the foot joints were subjected to a pathologic histological examination.

As a result, the hyperplasta of foot pad (>3.7 mm) was 6/14 cases (43%) for the treated group and 12/16 cases (75%) for the control group. Cases in which a histologically remarkable inflammatory cell infiltration was observed was 7/14 cases (50%) for the treated group, and 8/16 cases (50%) for the control group. The number of cases in which subjects had arthritis without damage was 12/14 cases (86%) for the treated group and 2/16 cases (13%) for the control group. The change in the body weight of mice between before and after experiment was 151% for the treated group and 144% for the control group: no difference was observed between the two groups. In a synovial culture system in vitro, while the administered AP-1 inhibited the expression system such as interleukin-1 and the like acting through AP-1 site, had no effect on gene expressions not passing through an AP-1 site: the specificity thereof was thus confirmed.

As is clear from the above-mentioned results, the administered AP-1 nucleotide significantly inhibited joint destruction of collagen-induced arthritis in the mice. In contrast to the significant inhibition of joint destruction, there was no difference in the extent of inflammatory cell infiltration to local legions of joints between the two groups. This result suggests that, in arthritis, inflammatory cell 1 in infiltration do not necessarily directly participates in joint destruction. This result is of interest, agreement with the result about the role of synovial mesenchymal cells in joint destruction with synovial mesenchymal cells in joint destruction with H2-c-fos transgenic mice.

### INDUSTRIAL APPLICABILITY

According to the present invention, as described above in detail, it is possible to treat collagen diseases particularly rheumatoid arthritis, thus permitting great expectation in clinical application. It is expected to apply the present invention for the clarification of mechanism and causes of rheumatoid arthritis, other chronic inflammatory diseases and intractable diseases of which proliferation of the cells of mesenchymal origin plays an important role in the pathogenesis and therapeutic application therefor.

## Claims

1. Use of a gene promoter sequence containing TGAGTCA or TGACTCA in the production of a medicament for the treatment of a collagen disease.

2. Use as claimed in claim 1 wherein the collagen disease is rheumatoid arthritis.

## Patentansprüche

1. Verwendung einer Gen-Promotorsequenz, enthaltend TGAGTCA oder TGACTCA, zur Herstellung eines Medikaments zur Behandlung einer Collagen-Krankheit.

2. Verwendung nach Anspruch 1, wobei die Collagen-Krankheit rheumatoide Arthritis ist.

## Revendications

1. Utilisation d'une séquence du promoteur d'un gêne contenant TGAGTCA ou TGACTCA pour la préparation d'un médicament destiné au traitement d'une connectivite.

2. Utilisation selon la revendication 1 dans laquelle la connectivite est la polyarthrite rhumatoïde.
